# EUROPÄISCHE PATENTSCHRIFT

(11) **EP 1 087 752 B1**
(45) Veröffentlichungstag und Bekanntmachung des Hinweises auf die Patenterteilung: **28.05.2008**
(21) Anmeldenummer: 99916882.6
(22) Anmeldetag: 24.03.1999
(51) Int. Cl.: A61K 9/127, A61K 31/7068

(54) **VERFAHREN ZUR HERSTELLUNG VON LIPOSOMALEN WIRKSTOFFFORMULIERUNGEN**
METHOD FOR PRODUCING LIPOSOMAL FORMULATIONS OF ACTIVE AGENTS
PROCEDE DE PREPARATION DE FORMULATIONS LIPOSOMALES DE PRINCIPES ACTIFS

(30) Priorität: 27.03.1998 DE 19813773
(43) Veröffentlichungstag der Anmeldung: 04.04.2001
(73) Patentinhaber: Unger, Clemens, 79104 Freiburg (DE); Massing, Ulrich, 79106 Freiburg (DE)
(72) Erfinder: UNGER, Clemens, D-79104 Freiburg (DE); MASSING, Ulrich, D-79106 Freiburg (DE); MOOG, Regina, D-78183 Hüfingen (DE)
(74) Vertreter: Dey, Michael
(86) Internationale Anmeldenummer: PCT/EP1999/001993
(87) Internationale Veröffentlichungsnummer: WO 1999/049716

(56) Entgegenhaltungen:
- WO-A-92/06676
- WO-A-92/10166
- WO-A-97/35560
- US-A- 5 616 341
- PONS M ET AL: "ENROFLOXACIN LOADED LIPOSOMES OBTAINED BY HIGH SPEED DISPERSION METHOD" CHEMICAL AND PHARMACEUTICAL BULLETIN, Bd. 43, Nr. 6, 1. Juni 1995 (1995-06-01), Seiten 983-987, XP000515136 ISSN: 0009-2363
- BAUER, KURT H.: "A simple preparatory method for liposomes and the experimental preparation of hemoglobin- and insulin-liposomes. Review of an experimental series on the preparation of parenterally and perorally applied liposomes" PHARM. UNSERER ZEIT, 1992, 21, 71-5, XP002114668
- TARDI C ET AL: "Erosion and controlled release properties of semisolid vesicular phospholipid dispersions." J CONTROLLED RELEASE, NOV 13 1998, 55 (2-3) P261-70, XP002114669 NETHERLANDS
- MOOG ET AL CANCER CHEMOTHER. PHARMACOL. 2002, Seiten 356 - 366

## Beschreibung

Die Erfindung betrifft ein Verfahren zur Herstellung von Liposomen, die als Wirkstoff Gemcitabine als eine physiologisch wirksame oder/und diagnostische Verbindung enthalten sowie die nach diesem Verfahren hergestellte Liposomenzubereitung.

Liposomen sind sphärische Gebilde, die aus Amphiphilen aufgebaut sind. In wässrigen Lösungen entstehen die Liposomen durch Selbstaggregation der Amphiphilen, wobei sich eine Lipiddoppelschicht bildet, die einen wässrigen Innenraum einschließt.

In Abhängigkeit von physikalischen Parametern wie Druck, Temperatur und Ionenkonzentration sowie der vorhandenen Lipide und Zusatzstoffe, bilden sich unilamellare, oligolamellare oder multilamellare Liposomen. Die Liposomen können abhängig von ihren Bestandteilen eine positive oder negative Überschussladung tragen.

Liposomen können auch mit Wirkstoffen beladen sein, die je nach Lipophilie oder Hydrophilie in der Lipidschicht oder im wässrigen Innenraum der Liposomen eingeschlossen sind. Derartige Liposomen werden in diagnostischen Nachweisverfahren oder als therapeutisches Mittel zum Transport von Wirkstoffen im Organismus oder als Wirkstoffdepot verwendet.

Die Eigenschaften der Liposomen wie beispielsweise ihre Stabilität oder Lagerfähigkeit werden wesentlich durch die in der Lipidschicht vorhandenen Substanzen bestimmt.

Zur Synthese von Liposomen werden membranbildende Lipide, wie beispielsweise Phosphatidylcholin, Phosphatidyglycerin, Phosphatidylinositol, Phosphatidylserin und Phosphatidylethanolamin, Sphingomyelin, membranbildende Amphiphile, wie beispielsweise Blockpolymere, Alkylester, -ether, -amide von Alkoholen, Diolen und Polyolen, Aminen, Aminosäuren, Peptiden und Zuckern und Cholesterin sowie weitere Stoffe verwendet (vgl. F. Szoka Jr. und D. Papahadjopoulos, Comperative Properties and Methods of Preparation of Lipidvesicles. (Liposomes), Ann. Rev. Biophys. Bioeng. 1980, 9:467-508; Liposomes: From Physical Strukture To Therapeutic Application (1981), Knight, C.G. (ED.), Elsevier, North Holland Biomedical Press, Kapitel 2: H. Eibel, Phospholipidssynthesis, 19-50; Kapitel 3: F. Szoka und D. Papahadjopoulos, Liposomes: Preparation and Characterization, 51-104).

Zur Herstellung von Wirkstoffe enthaltende Liposomen sind mechanische Verfahren bekannt, bei denen eine die Membranbestandteile und Wirkstoffe enthaltende Dispersion durch Einwirkung starker mechanischer Kräfte, z.B. Hochdruckhomogenisation z.B. mittels einer French-Press behandelt werden. Ein anderes Verfahren basiert auf dem Austausch des organischen Lösungsmittels durch wässriges Medium oder auf der einfachen Entfernung des in der Dispersion enthaltenen Detergenz. Ein weiteres Verfahren zur Liposomenherstellung beruht auf der Veränderung des pH-Wertes (vgl. F. Szoka Jr. und D. Papahadjopoulos)

Aufgrund der Eigenschaften der einzuschließenden Wirkstoffe ist es in vielen Fällen für die Herstellung von Wirkstoffe enthaltenden Liposomen notwendig, dass die Liposomenbildung und der Einschluss der Wirkstoffe in nur einem Verfahrensschritt vollzogen wird. Die Liposomenformation und Wirkstoffeinschluss in einem Schritt ist aber oft nicht durchführbar bzw. kann verschiedene Nachteile mit sich bringen: So kann es z.B. unter den Bedingungen der Hochdruckhomogenisation (French Press, Gaulin ..) durch die Wechselwirkungen zwischen dem Wirkstoff und den eingesetzten Lipiden und dem Medium zur Zerstörung des Wirkstoffs, zur Hydrolyse der Lipide oder zu beidem kommen. Aus gleichen Gründen ist oft eine Sterilisierung der wirkstoffhaltigen Formulierung nicht möglich, es werden Lipidhydrolyse und/oder Zerstörung des Wirkstoffs beobachtet. Eine alternative Sterilfiltration zur Sterilisierung ist bei der Herstellung von Liposomen-Gelen nicht möglich, da diese zu hoch viskos sind. Auch bei Liposomendispersionen ist eine Sterilfiltration oft aufgrund der Größe der Vesikel nicht möglich.

Des Weiteren ist die Erarbeitung eines bestimmten Liposomen-Gels oder einer Liposomendispersion ohne gleichzeitiges Zusetzen des Wirkstoffs nicht möglich, da Wirkstoffe oft entscheidend die Formation der Lipidbilayer beeinflussen und so auch Einfluss auf die Größe der entstehenden Vesikel haben können.

Bedingt durch die apparativen Voraussetzungen bei der Liposomenherstellung ist oft die Herstellung einer Mindestmenge einer liposomalen Formulierung notwendig, die die für die Untersuchung der Präparation tatsächlich notwendige Menge weit überschreitet. Dies ist insbesondere dann von Nachteil, wenn der für den Einschluss verwendete Wirkstoff nur in begrenzter Menge zur Verfügung steht oder sehr teuer ist.

Somit können nur die Wirkstoffe enthaltenden Liposomen auf ihre Charakteristika untersucht werden. Zeigt die so hergestellte Liposomenpräparation nicht die gewünschten Eigenschaften, so muss diese Präparation einschließlich der darin enthaltenen kostbaren Wirkstoffe als Ganzes verworfen werden. Dies ist insbesondere dann von Nachteil, wenn der für die Einkapselung verwendete Wirkstoff nur in begrenzter Menge zur Verfügung steht oder sehr teuer ist.

Für bestimmte Anwendungen von Liposomenzubereitungen, insbesondere bei einer therapeutischen Anwendung, ist es außerdem erforderlich, die Liposomenpräparation zu sterilisieren. Im allgemeinen bedient man sich dazu der Sterilisation durch Autoklavieren, bei der erhöhte Temperatur- und Druckbedingungen auftreten. Bestimmte in Liposomen eingeschlossene Substanzen bewirken jedoch bei erhöhter Temperatur eine Phospholipidhydrolyse, sodass es bei einer derartigen Behandlung zur Zersetzung der Liposomenpräparation kommt. Außerdem können die Wirkstoffe bei erhöhter Temperatur instabil sein und sich beim Autoklavieren zersetzen. Somit kann die Sterilisation durch Autoklavieren für eine derartige Liposomenpräparation nicht angewendet werden.

Ein weiterer Nachteil bei der Herstellung einer wirkstoffhaltigen Liposomenformulierung durch Hochdruckhomogenisastion ist die mögliche Aerosolbildung, die bei der Verwendung bestimmter Wirkstoffe eine erhebliche Gefährdung für das Bedienungspersonal darstellt. Die erforderlichen Sicherheitsvorkehrungen sind aufwendig und erschweren den Herstellungsprozess. Dies führt zu einem größeren Zeitaufwand, vermehrtem Einsatz von Arbeitsmitteln und einem höheren Personalaufwand, wodurch die Herstellungskosten erheblich erhöht werden.

Aus der US 5,616,341 ist ein aktiver Beladungsmechanismus von Liposomen über einen Transmembran-pH-Gradienten bekannt. Hierzu werden spezielle Puffer eingesetzt, die dann auch in den fertigen Liposomen vorhanden sind. Eine passive Beladung ist nicht offenbart

In der WO 92/06676 ist ein Verfahren zur Herstellung von ölgefüllten paucilamellaren Lipidvesikeln beschrieben, das nur für einen sehr speziellen Liposomentyp geeignet ist. Dabei werden unpolare Substanzen in den Zentralhohlraum der Liposomen eingeschlossen. Man erhält somit lipidumhüllte Öltropfen. Das Verfahren eignet sich somit nur für lipophile Verbindungen und die Abtrennung der Vesikel vom nicht inkorporierten Material wird als essentiell angesehen.

WO 92/10166 beschreibt ein Verfahren zur Herstellung von Liposomen mit erhöhter Einschlusskapazität, wobei ein möglichst, vollständiger und endgültiger Einschluss der Wirkstoffe angestrebt wird. Der Einschlussvorgang soll nicht reversibel sein. Der nicht verkapselte Wirkstoff wird abgetrennt.

In der WO 97/35560 wird ein-Verfahren zur Herstellung von Liposomen durch Einschluss von amphiphilen Verbindungen beschrieben, wobei sogenannte Stealth-Liposomen verwendet werden. Freier Wirkstoff muss vom beladenen Liposom abgetrennt werden.

Aus M. Pons, Chem. Pharm. Bull. 43(6), 1995, (983-987) ist .ein einstufiges Verfahren zur Liposomenherstellung bekannt, so dass Liposomenbildung und Einschluss der Wirkstoffe in einem einzigen Verfahrensschritt erfolgen. Unter den Bedingungen der Hochdruckhomogenisation treten beim einstufigen Verfahren Wechselwirkungen zwischen Wirkstoff und Lipid auf unter Zerstörung des Wirkstoffs, Hydrolyse der Lipide oder beidem. Freier, nicht eingeschlossener Wirkstoff wird abgetrennt.

Die der Erfindung zugrundeliegende Aufgabe war es somit, ein Verfahren zur Herstellung von Gemcitabine als Wirkstoff enthaltenden Liposomen bereitzustellen, mit dem sterile Liposomenpräparationen kostengünstig und ohne Gefährdung des Bedienungspersonals bereitgestellt und die Nachteile des Standes der Technik zumindest teilweise verbessert werden können.

Diese Aufgabe wird gelöst durch ein Verfahren zur Herstellung von Liposomen, die Gemcitabine als eine physiologisch wirksame oder/und diagnostische Verbindung enthalten, durch Herstellen eines Gemisches von membranbildenden Amphiphilen, Dispergieren des Gemisches in Wasser und Hochdruckhomogenisation der Dispersion bis zur Bildung der Liposomen, das dadurch gekennzeichnet ist, dass
(a) zu einer beliebigen Liposomenzubereitung, vorzugsweise ein Liposomen-Gel, das mindestens 20 Gew.-% Lipid enthält, der Wirkstoff Gemcitabine gegeben wird und
(b) das den Wirkstoff Gemcitabine enthaltende Gemisch, gegebenenfalls unter mechanischer Bewegung, solange inkubiert wird, bis
   (i) der hydrophile Wirkstoff entsprechend der Volumenanteile an wässerigem Medium innerhalb und außerhalb der Liposomen in der Zubereitung in den Liposomen bzw: zwischen den Liposomen verteilt ist (Gleichgewichtszustand).

Beispielhaft wird ein Verfahren zur Herstellung von Liposomen beschrieben, die als Wirkstoffe mindestens eine physiologisch wirksame oder/und diagnostische Verbindung enthalten, wobei das Verfahren die Herstellung eines Gemisches von membranbildenden Amphiphilen, das Dispergieren des Gemisches in Wasser und eine Hochdruckhomogenisation der Dispersion bis zur Bildung der Liposomen umfasst,
dadurch gekennzeichnet, dass
(a) zu einer beliebigen Liposomenzubereitung, vorzugsweise ein Liposomen-Gel, das mindestens 20 Gew.% Lipid enthält, der Wirkstoff gegeben wird und
(b) das den Wirkstoff enthaltende Gemisch, gegebenenfalls unter mechanischer Bewegung solange inkubiert wird, bis
   (i) lipophile Verbindungen im Lipidbilayer inkorporiert sind, oder
   (ii) amphiphile Verbindungen den Lipidbilayer überwunden haben und in den äußeren Regionen des Lipidbilayers inkorporiert bzw. assoziiert sind.

Hierbei wird die Temperatur so geregelt, dass keine wesentliche Lipidhydrolyse auftritt, die Diffusion der Wirkstoffe über den Lipidbilayer bzw. die Aufnahme in den Bilayer aber gesteigert wird.

Als hydrophiler Wirkstoff wird erfindungsgemäß Gemcitabine eingesezt. Beispielhaft können als Wirkstoffe alle Verbindungen eingesetzt werden, die unter den gegebenen Bedingungen. über Lipidbilayer diffundieren (hydrophile Verbindungen) oder in Lipidbilayer inkorporiert werden können (lipophile Verbindungen).

Es war überraschend, dass mit dem erfindungsgemäßen Verfahren eine fertige Liposomenzubereitung nachträglich mit guten Ausbeuten mit Wirkstoffen beladen werden kann. Unerwartet war, dass der Wirkstoff als Feststoff oder in konzentrierter wässriger Lösung zu der fertigen Liposomenzubereitung zugegeben werden kann und durch Temperaturerhöhung und gegebenenfalls durch einfaches mechanisches Durchmischen, Rühren oder Schütteln unter Verwendung einer für diesen Zweck geeigneten Vorrichtung, wie z.B. eines Horizontalschüttlers, zu guten Einschlussraten des Wirkstoffs in die Liposomenzubereitung führt. Ein weiterer Vorteil ist die einfache Durchführbarkeit des erfindungsgemäßen Verfahrens sowie der geringe apparative und personelle Aufwand. Mit dem erfindungsgemäßen Verfahren wird auch die Einkapselung von toxischen Stoffen ohne besondere Sicherheitsmaßnahmen und dem Einsatz von aufwendigen Sicherheitsvorkehrungen apparativer Art möglich. Die Gefährdung des Bedienungspersonals durch Aerosolbildung ist im erfindungsgemäßen Verfahren im Gegensatz zu bekannten Verfahren weitgehend ausgeschlossen. Das erfindungsgemäße Verfahren führt somit zu einer erheblichen Kostenreduzierung und Vereinfachung des Herstellungsprozesses für Wirkstoffe enthaltende Liposomenzubereitungen.

In dem erfindungsgemäßen Verfahren können alle bekannten Liposomenzubereitungen, wie beispielsweise unilamellare, bilamellare, multilamellare Liposomenzubereitung oder/und Liposomen mit positiver oder negativer Überschussladung verwendet werden.

Als Bestandteile der Lipidschicht der Liposomen können alle für diesen Zweck geeigneten membranbildenden Amphiphile sowie weitere Stoffe verwendet werden, wie beispielsweise Phosphatidylcholin, Phosphatidylglycerin, Phosphatidylinositol, Phosphatidylserin, Phosphatidylethanolamin, Sphingomyelin, Cholesterin, gesättigte und ungesättigte Fettsäuren, membranbildende Amphiphile, wie beispielsweise Blockpolymere, Alkylester, -ether, -amide von Alkoholen, Diolen, Polyolen, Aminen, Aminosäuren, Peptiden und Zuckern sowie weitere Stoffe, die sich in einer wässrigen Umgebung in der Lipidschicht anordnen. Gegebenenfalls können organische oder anorganische Salze sowie Säuren oder Basen zur Einstellung des pH-Wertes und des osmotischen Drucks in der Liposomenzubereitung gelöst sein. Insbesondere bei Verwendung der nach dem erfindungsgemäßen Verfahren hergestellten Liposomenzubereitung für medizinische Zwecke, wie beispielsweise Injektion oder Einbringen der erfindungsgemäßen Liposomenzubereitung in Körperhöhlen werden sterile Lösungen und Substanzen sowie Wasser nach Deutschem Arzneibuch ("Wasser für Injektionszwecke") verwendet. Die Liposomenzubereitung kann vor der Inkubation mit dem Wirkstoff durch geeignete Methoden,sterilisiert werden, wie beispielsweise autoklavieren.

Die Liposomen der Liposomenzubereitung können kleine, mittlere oder große Durchmesser aufweisen, vorzugsweise wird eine Liposomenzubereitung mit Liposomen mit einem durchschnittlichen Durchmesser von 10 bis 400 nm verwendet.

Die Liposomenzubereitung kann eine Liposomen enthaltende Dispersion sein, vorzugsweise wird ein aus Liposomen aufgebautes Gel verwendet.

Für das erfindungsgemäße Verfahren wird Gemcitabine verwendet, welches aufgrund seiner Diffusionseigenschaften in der Lage ist, (langsam) in oder über den Lipidbilayer zu diffundieren. Es kann ein einziger Wirkstoff eingeschlossen werden oder auch eine Mehrzahl davon. Der Einschluss kann im Innenraum der Liposomen oder/und in der Membran oder/und zwischen Membranschichten erfolgen. Alle diese Einschlussarten werden zusammenfassend als Inkorporation bezeichnet.

Als in der Forschung eingesetzter Fluoreszenzfarbstoff wird vorzugsweise Calcein verwendet.

Die Verfahrensbedingungen werden an die jeweils verwendete Liposomenzubereitung und den verwendeten Wirkstoff angepasst, um die gewünschte Aufnahme des Wirkstoffs in die Liposomenzubereitung zu erhalten. Die Aufnahmegeschwindigkeit und Einschlusseffizienz des Wirkstoffs in die Liposomenzubereitung werden durch die Diffusionseigenschaften des Wirkstoffs und die Liposomencharakteristika beeinflusst.

Die Diffusionseigenschaften des Wirkstoffs sind wiederum abhängig von Parametern, wie beispielsweise Molgewicht, Öl-Wasser-Koeffizient oder Protonierungsgrad.

Die Liposomencharakteristika werden bestimmt durch Parameter, wie beispielsweise Art und Gehalt der membranbildenden Amphiphilen, Gehalt an Cholesterin, und dem Gehalt und der Art von zusätzlichen Stoffen in der Lipidschicht. Insbesondere das maximale Verhältnis von wässriger Phase in den Liposomen zu wässriger Phase außerhalb der Liposomen sowie Vorliegen der Liposomen in einer Liposomendispersion oder in einem Liposomengel bestimmen die Einschlusseffizienz.

Alle für das Verfahren relevanten Parameter werden optimal aufeinander abgestimmt. Insbesondere die Temperatur wird so eingestellt, dass die Diffusion des Wirkstoffs in die Liposomen gesteigert wird, ohne dass es zu einer wesentlichen Phospholipidhydrolyse kommt. Vorzugsweise wird das den Wirkstoff enthaltende Gemisch bei einer Temperatur von 30 bis 80°C, mehr bevorzugt von 50-70°C und am meisten bevorzugt um etwa 60°C inkubiert. Zeigt das Lipidgemisch eine Phasenumwandlungstemperatur oberhalb Raum- bzw. Lagertemperatur, wird vorzugsweise über diese Temperatur erwärmt.

Inkubationstemperatur und Inkubationszeit hängen voneinander ab, d.h. bei Erhöhung der Inkubationstemperatur kann die Inkubationszeit vermindert werden, will man den gleichen Einschlussgrad wie bei verminderter Inkubationstemperatur erhalten.

Die einzelnen Komponenten werden solange inkubiert, bis die maximale Aufnahme erreicht ist (Gleichgewichtszustand bei hydrophilen Verbindungen, nahezu vollständige. Aufnahme bei lipophilen Verbindungen). Bei einer nicht vollständigen Aufnahme des Wirkstoffs in die Liposomen des Gels befindet sich somit ein Teil des Wirkstoffs in den Liposomen und der übrige zu der Liposomenzubereitung zugegebene Wirkstoff außerhalb der Liposomen in der Suspension oder dem Gel. Ein so hergestelltes Liposomen-Gel kann den Wirkstoff in verzögerter Weise über einen längeren Zeitraum abgeben, wobei eine gute Retardwirkung dadurch erreicht wird, dass ein Wirkstoff, um das Liposomengel zu verlassen, vielfach aus einzelnen Liposomen herausdiffundieren und wiederum in Liposomen hineindiffundieren muss, da die Liposomen sehr dicht gepackt sind. Andererseits kann bei auf die erfindungsgemäße Weise hergestellten Liposomendispersionen der nicht in die Liposomen eingeschlossene Wirkstoff rasch zur Wirkung gelangen (Initialdosis), während der eingeschlossene Anteil verzögert freigesetzt und wirksam wird. Alternativ wird der nicht inkorporierte Teil des Wirkstoffs aus der Liposomenzubereitung abgetrennt, wenn freier Wirkstoff nicht erwünscht ist. Vorzugsweise wird ein erfindungsgemäß hergestelltes Wirkstoffe enthaltendes Liposomengel für medizinische Applikationen verwendet.

Wie oben beschrieben, hängt die Aufnahmegeschwindigkeit und Einschlusseffizienz unter anderem von den Eigenschaften des Wirkstoffs ab. Lipophile und amphiphile Wirkstoffe werden von den Liposomen im wesentlichen in der Lipidschicht eingeschlossen, wogegen hydrophile Wirkstoffe im wesentlichen in dem wässrigen Innenraum der Liposomen eingeschlossen werden. Lipophile Wirkstoffe zeigen sehr hohe Einschlussraten. Sie können mit einer Einschlussrate von bis zu 100 % in Liposomen inkorporiert werden. Amphiphile Verbindungen, welche an und in die äußeren Membranbezirke inkorporieren bzw. angelagert werden, führen zu Einschlussraten von bis zu 50%. Bei Liposomen, die aus mehreren Bilayern aufgebaut sind, kann die Einschlussrate höher liegen. Zum Beispiel kann das Gemisch inkubiert werden, bis der lipophile Wirkstoff vollständig in die Liposomenmembran aufgenommen ist.

Ein weiterer Gegenstand der Erfindung ist eine Liposomenzubereitung, erhalten bzw. erhältlich nach einem wie oben beschriebenen Verfahren.

Eine. Liposomenzubereitung, erhalten bzw. erhältlich nach einem wie oben beschriebenen Verfahren, kann als diagnostisches oder therapeutisches Mittel, das gegebenenfalls weitere Hilfs-, Trägerstoffe oder/und Stabilisatoren wie z.B. Zucker, reduzierte Zucker und Polyalkohole enthält, verwendet werden.

Ein weiterer Gegenstand der Erfindung ist eine Liposomenzubereitung, erhalten bzw. erhältlich durch Redispergieren der Liposomen durch schrittweise Zugabe von wässrigem Medium zu dem die Liposomen enthaltenden Gel. Die so erhaltene Formulierung kann systemisch, beispielsweise durch i.v. Injektion appliziert werden. Nicht eingeschlossener Wirkstoff kann durch geeignete Verfahren abgetrennt werden.

Die folgenden Beispiele sollen die Erfindung weiter erläutern.

### Beispiele

### Beispiel 1

Ein Liposomengel, das als Wirkkomponente das Zytostatikum Gemcitabine enthält, kann nicht autoklaviert werden, da dieses Antitumormittel die Hydrolyse der Phospholipide beschleunigt, was unter Standardbedingungen während des Autoklavierens zu einem vollständigem Phospholipidabbau führt. Die Anforderung der I<eimfreiheit für Arzneimittel zur parenteralen Anwendung kann nur durch nachträgliches Einbringen des Wirkstoffes in das Liposomengel erreicht werden. Hierzu werden das Liposomengel und eine hochkonzentrierte, gepufferte Gemcitabine-Lösung getrennt autoklaviert und anschließend unter aseptischen Bedingungen zusammengebracht. Nach vierstündiger Inkubation bei 60°C wird eine Schlusseffizienz von über 30% erreicht (Gleichgewicht).

Diese Zubereitung eignet sich zur direkten Installation in die Bauchhöhle bei Ovarial-CA; zur Blasenspülung bei Blasen-CA sowie zur intratumoralen Applikation, nach Redispergierung auch zur i.v. Applikation.

### Beispiel 2 (Vergleichsbeispiel)

Das Zytostatikum Vindesine ist hochwirksam und wird in der Klinik in niedrigen Gesamtdosen eingesetzt. Wird der Wirkstoff mittels der herkömmlichen Technik der Liposomengelherstellung bereits während der Hochdruckhomogenisation eingeschlossen, sind strenge Sicherheitsmaßnahmen zu treffen, um eine Inhalation entstehender Aerosole und mögliche Spätschäden des Bedienungspersonals zu verhindern. Zudem ist Vincristine unter den Bedingungen der Hochdruckhomogenisation oder des Autoklavierens nicht stabil. Das nachträgliche Einbringen der Substanz vermindert die Aerosolbildung wesentlich und vermindert somit das Arbeitsrisiko bedeutend. Die Herstellung erfolgt entsprechend Beispiel 1.

Auch diese Formulierung kann lokal zur Behandlung von Lungen-CAs eingesetzt werden und nach Redispergierung i.v. appliziert werden.

### Beispiel 3 (Vergleichsbeispiel)

Der Fluoreszenzmarker Calcein wird häufig in der Grundlagenforschung eingesetzt. Er ist nicht autoklavierbar, sodass sterile Calcein-Liposomengele nur durch nachträglichen Calcein-Zusatz, hergestellt werden können. Die Herstellung erfolgt entsprechend Beispiel 1 nach Sterilfiltration der Calcein-Lösung. Mittels dieser Zubereitung lassen sich Freisetzungs- und Verteilungsversuche, z.B. in der Mikrobiologie durchführen.

### Beispiel 4 (Vergleichsbeispiel)

Das Verfahren eignet sich grundsätzlich auch zur Verkapselung von lipophilen Substanzen, die nicht in das wässerige Kompartiment, sondern in den Liposomenlayer eingeschlossen werden. Hierzu wird das Antibiotikum Amphotericin B als Festsubstanz dem Liposomengel zugesetzt und anschließend bei 50°C für 3 Stunden unter Schütteln inkubiert. Die Einschlusseffizienz beträgt 95 %.

## Patentansprüche

1. Verfahren zur .Herstellung von Liposomen, die Gemcitabine als Wirkstoff enthalten, durch Bildung einer Mischung von membranbildenden Amphiphilen, Dispergieren dieser Mischung in Wasser und Hochdruckhomogenisation der Dispersion unter Liposomenbildung, wobei
(a) der Wirkstoff Gemcitabine den Liposomen zugesetzt und
(b) die den Wirkstoff enthaltende Mischung inkubiert wird, bis der hydrophile Wirkstoff in den Liposomen und zwischen den Liposomen entsprechend den Volumenanteilen des wäßrigen Mediums innerhalb und außerhalb der Liposomen im Präparat verteilt ist (Gleichgewichtszustand).

2. Verfahren nach Anspruch 1, **dadurch gekennzeichnet, dass** ein Liposomengel als lipidhaltige Liposomenpräparation verwendet wird.

3. Verfahren nach Anspruch 2, **dadurch gekennzeichnet, dass** ein Liposomengel mit einem Lipidgehalt von mindestens 20 Gew.% verwendet wird.

4. Verfahren nach einem der Ansprüche 1-3, **dadurch gekennzeichnet, dass** die Inkubation unter mechanischer Bewegung erfolgt.

5. Verfahren nach Anspruch 1, **dadurch gekennzeichnet, dass** eine liposomenhaltige Dispersion verwendet wird.

6. Verfahren nach einem der Ansprüche 2 oder 3, **dadurch gekennzeichnet, dass** die Liposomen redispergiert werden, indem wässriges Medium schrittweise dem liposomhaltigen Gel zugesetzt wird.

7. Verfahren nach Anspruch 6 zur Herstellung einer Formulierung, die systemisch verabreicht werden kann.

8. Verfahren nach einem der vorhergehenden Ansprüche, **dadurch gekennzeichnet, dass** die den Wirkstoff enthaltende Mischung bei einer Temperatur von 30-80 °C, vorzugsweise 50-70 °C, besonders bevorzugt von 60 °C inkubiert wird.

9. Liposomenpräparation erhältlich nach dem Verfahren nach einem der Ansprüche 1 bis 8.

10. Zusammensetzung zur Herstellung einer Liposomenpräparation nach Anspruch 5, **dadurch gekennzeichnet, dass** sie als Komponente A durch Hochdruckhomogenisation hergestellte Liposomen und als Komponente B Gemcitabine als Feststoff oder in konzentrierter wässriger Lösung enthält.

11. Zusammensetzung nach Anspruch 10; **dadurch gekennzeichnet, dass** die Komponente A ein Liposomengel und die Komponente B eine hochkonzentrierte gepufferte Gemcitabine-Lösung umfasst, und dass die Komponenten A und B getrennt autoklaviert werden.

## Claims

1. Process for producing liposomes which contain gemcitabine as an active substance by forming a mixture of membrane-forming amphiphiles, dispersing this mixture in water and high pressure homogenization of the dispersion to form liposomes wherein
(a) the active substance gemcitabine is added to the liposomes and
(b) the mixture containing the active substance is incubated until the hydrophilic active substance is distributed in the liposomes and between the liposomes according to the proportions by volume of the aqueous medium inside and outside the liposomes in the preparation (equilibrium state).

2. Process according to claim 1, **characterized in that** a liposome gel is used as a lipid-containing liposome preparation.

3. Process according to claim 2, **characterized in that** a liposome gel with a lipid content of at least 20 % by weight is used.

4. Process according to one of the claims 1 to 3, **characterized in that** the incubation takes place with mechanical agitation.

5. Process according to claim 1, **characterized in that** a dispersion containing liposomes is used.

6. Process according to one of the claims 2 or 3, **characterized in that** the liposomes are redispersed by the stepwise addition of aqueous medium to the gel containing liposomes.

7. Process according to claim 6 for producing a formulation which can be administered systemically.

8. Process according to one of the previous claims, **characterized in that** the mixture containing the active substance is incubated at a temperature of 30 - 80°C, preferably 50 - 70°C and particularly preferably at 60°C.

9. Liposome preparation obtainable by a process according to one of the claims 1 to 8.

10. Composition for producing a liposome preparation according to claim 5, **characterized in that** it contains liposomes produced by high pressure homogenization as component A and gemcitabine as a solid substance or in a concentrated aqueous solution as component B.

11. Composition according to claim 10, **characterized in that** component A comprises a liposome gel and component B comprises a highly concentrated buffered gemcitabine solution, and that components A and B are autoclaved separately.

## Revendications

1. Procédé de fabrication de liposomes contenant de la gemcitabine comme principe actif, par constitution d'un mélange de composés amphiphiles formant des membranes, dispersion de ce mélange dans l'eau et homogénéisation sous haute pression de la dispersion de manière à former des liposomes, dans lequel
(a) le principe actif gemcitabine est ajouté aux liposomes et
(b) le mélange contenant le principe actif est incubé jusqu'à ce que le principe actif hydrophile dans les liposomes soit réparti et entre les liposomes de façon correspondante à la proportion en volume du milieu aqueux à l'intérieur et à l'extérieur des liposomes dans la préparation (état d'équilibre).

2. Procédé selon la revendication 1, **caractérisé en ce qu'**un gel liposomique est utilisé comme préparation liposomique contenant des lipides.

3. Procédé selon la revendication 2, **caractérisé en ce qu'**un gel liposomique ayant une teneur en lipides d'au moins 20 % en poids est utilisé.

4. Procédé selon l'une quelconque des revendications 1 à 3, **caractérisé en ce que** l'incubation s'effectue sous un mouvement mécanique.

5. Procédé selon la revendication 1, **caractérisé en ce qu'**une dispersion liposomique est utilisée.

6. Procédé selon l'une quelconque des revendications 2 ou 3, **caractérisé en ce que** les liposomes sont redispersés par addition progressive d'un milieu aqueux au gel contenant les liposomes.

7. Procédé selon la revendication 6 pour fabriquer une formulation pouvant être administrée de manière systémique.

8. Procédé selon l'une quelconque des revendications précédentes, **caractérisé en ce que** le mélange contenant le principe actif est incubé à une température de 30 à 80 °C, de préférence de 50 à 70 °C, de manière particulièrement préférée de 60 °C.

9. Préparation de liposomes susceptible d'être obtenue par le procédé selon l'une quelconque des revendications 1 à 8.

10. Composition pour fabriquer une préparation liposomique selon la revendication 5, **caractérisée en ce qu'**elle contient, comme composant A, des liposomes obtenus par homogénéisation sous haute pression et, comme composant B, de la gemcitabine sous forme de substance solide ou en solution aqueuse concentrée.

11. Composition selon la revendication 10, **caractérisée en ce que** le composant A comprend un gel liposomique et le composant B comprend une solution de gemcitabine hautement concentrée et **en ce que** les composants A et B sont autoclavés séparément.
